# EUROPÄISCHE PATENTSCHRIFT

(11) **EP 1 501 859 B1**
(45) Veröffentlichungstag und Bekanntmachung des Hinweises auf die Patenterteilung: **26.10.2005**
(21) Anmeldenummer: 03725149.3
(22) Anmeldetag: 05.05.2003
(51) Int. Cl.: C07J 75/00

(54) **17a-FLUORALKYL-11BETA-BENZALDOXIM-STEROIDE, VERFAHREN ZU DEREN HERSTELLUNG, DIESE STEROIDE ENTHALTENDE PHARMAZEUTISCHE PRÄPARATE SOWIE DEREN VERWENDUNG ZUR HERSTELLUNG VON ARZNEIMITTELN**
17a-FLUOROALKYL-11BETA-BENZALDOXIME-STEROIDS, METHOD FOR THE PRODUCTION THEREOF, PHARMACEUTICAL PREPARATIONS CONTAINING SAID STEROIDS AND THE USE THEREOF IN THE PRODUCTION OF MEDICAMENTS
STEROIDES CONSTITUES DE 17a-FLUOROALKYL-11BETA-BENZALDOXIME, LEUR PROCEDE DE PREPARATION, PREPARATIONS PHARMACEUTIQUES CONTENANT LESDITS STEROIDES AINSI QUE LEUR UTILISATION POUR LA PRODUCTION DE MEDICAMENTS

(30) Priorität: 03.05.2002 DE 10221034; 25.02.2003 US 449401 P
(43) Veröffentlichungstag der Anmeldung: 02.02.2005
(73) Patentinhaber: Schering AG, 13353 Berlin (DE)
(72) Erfinder: RING, Sven, 07749 Jena (DE); SCHUBERT, Gerd, 07743 Jena (DE); TORNUS, Ingo, 16761 Hennigsdorf (DE); KAUFMANN, Günter, 07743 Jena (DE); ELGER, Walter, 14195 Berlin (DE); SCHNEIDER, Birgitt, 07745 Jena (DE)
(74) Vertreter: Ziebig, Marlene
(86) Internationale Anmeldenummer: PCT/EP2003/004686
(87) Internationale Veröffentlichungsnummer: WO 2003/093292

(56) Entgegenhaltungen:
- EP-A- 0 909 764
- DE-A- 19 706 061

## Beschreibung

Die vorliegende Erfindung betrifft neuartige 17α-Fluoralkyl-11β-benzaldoxim-Steroide, ein Verfahren zu deren Herstellung, diese Wirkstoffe enthaltende pharmazeutische Präparate sowie deren Verwendung zur Herstellung von Arzneimitteln, insbesondere zur postmenopausalen Substitutionstherapie von gynäkologischen Erkrankungen, wie Uterusmyomen oder dysmenorischen Beschwerden.

Antigestagen wirksame Steroide sind bereits aus EP 0 057 115 A2 bekannt. Es kann sich hierbei um in 11-Stellung substituierte 3-Oxo-estra-4,9-diene handeln.

Aus DE 43 32 283 A1, DE 43 32 284 A1, DE 198 09 845 A1 (WO 9945023 A1) sind 11 β-Benzaldoxime der Steroidreihe bekannt, die spezielle antigestagene Eigenschaften aufweisen:

In DE 43 32 283 A1 und DE 43 32 284 A1 sind 11 β-Benzaldoxim-3-oxo-estra-4,9-dien-Derivate beschrieben, die gemäß DE 43 32 283 A1 in 17β-Stellung mit Hydroxy, Alkoxy, Acyloxy oder Aryloxy und in 17α-Stellung mit ω-Fluoralkyl substituiert sein können.

In DE 198 09 845 A1 sind substituierte 11β-Benzaldoxim-3-oxo-estra-4,9-diene beschrieben. Es handelt sich hierbei um S-substituierte Kohlensäurethiolester dieser Verbindungen. Die Verbindungen können ebenfalls in 17β-Stellung mit Hydroxy, Alkoxy, Acyloxy oder Aryloxy und in 17α-Stellung mit ω-Fluoralkyl substituiert sein.

Andererseits sind Steroide mit 17α-Fluoralkylketten in DE 197 06 061 A1 offenbart. Diese Verbindungen, insbesondere ZK 230211 (11 β-(4-Acetylphenyl)-17β-hydroxy-17α-(1,1,2,2,2-pentafluorethyl)-estra-4,9-dien-3-on): U.Fuhrmann; H.Hess-Stumpp, A.Cleve, G.Neef, W.Schwede, J.Hoffmann, K.-H.Fritzemeier und K.Chwalisz: *J. Med. Chem.,* 2000, 43, 5010-5016), zeigen eine nahezu reine antagonistische Aktivität, hohe Rezeptorselektivität und u.a. antiproliferative Aktivität in Tumormodellen.

Die der vorliegenden Erfindung zugrunde liegende Aufgabe besteht darin, Wirkstoffe mit antigestagener Wirkung zu finden, die gegenüber den bekannten Verbindungen deutlich reduzierte antiglucocorticoide Wirkung aufweisen und die zur postmenopausalen Substitutionstherapie oder zur Behandlung von gynäkologischen Erkrankungen, wie Uterusmyomen oder dysmenorischen Beschwerden geeignet sind.

Eine weitere Aufgabe, die der vorliegenden Erfindung zugrunde liegt, besteht darin, ein Verfahren zur Herstellung der Wirkstoffe zu finden.

Weiterhin besteht eine Aufgabe darin, pharmazeutische Präparate zu finden, die die Wirkstoffe enthalten.

Die Aufgabe wird gelöst durch die erfindungsgemäßen 17α-Fluoralkyl-11β-benzaldoxim-Steroide gemäß Anspruch 1, durch das Verfahren zu deren Herstellung gemäß Anspruch 7, durch ein pharmazeutisches Präparat, enthaltend die erfindungsgemäßen Verbindungen gemäß Anspruch 10 sowie die Verwendung der erfindungsgemäßen Verbindungen zur Herstellung von Arzneimitteln gemäß Anspruch 11. Bevorzugte Ausführungsformen der Erfindung sind in den Unteransprüchen angegeben.

Die erfindungsgemäßen Verbindungen haben die allgemeine Formel **I:** 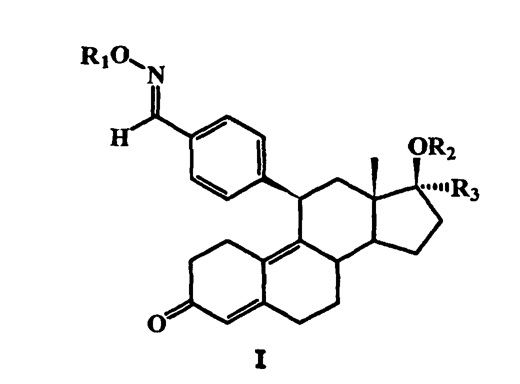 worin
**R**_{**1**} für Wasserstoff, C₁- bis C₆-Alkyl, COR₄, COOR₄, COSR₄ oder CONHR₅ steht, worin **R**_{**4**} C₁- bis C₆-Alkyl oder unsubstituiertes oder substituiertes Aryl ist und worin **R**_{**5**} Wasserstoff, C₁- bis C₆-Alkyl oder unsubstituiertes oder substituiertes Aryl ist,
**R**_{**2**} für Wasserstoff, C₁- bis C₆-Alkyl oder C₁- bis C₆-Acyl steht und
**R**_{**3**} für eine CₙF₂ₙ₊₁-Gruppe, bei der n = 1, 2 oder 3 ist, oder für eine CH₂O(CH₂)ₘCₙF₂ₙ₊₁-Gruppe steht, bei der m = 0 oder 1 und n = 1, 2 oder 3 sind.

In allen übrigen Positionen des Steroidgrundgerüstes sowie an dem Phenylenrest in 11β-Stellung können anstelle von Wasserstoff andere beliebige Substituenten, insbesondere Alkyl- und Arylgruppen, gebunden sein. Außerdem betrifft die vorliegende Erfindung pharmazeutisch verträgliche Salze dieser Verbindungen. Derartige Säureadditionssalze können Salze anorganischer und organischer Säuren sein, beispielsweise Salze der Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beispielsweise in: *Fortschritte der Arzneimittelforschung*, Bd. 10, Seiten 224-225, Birkhäuser Verlag, Basel und Stuttgart, 1966 sowie in: *Joumal of Pharmaceutical Sciences,* Bd. 66, Seiten 1-5 (1977) beschrieben.

Bei den in der vorliegenden Erfindung erwähnten Resten **R**_{**1**} handelt es sich insbesondere um ein Wasserstoffatom oder um eine Methylgruppe oder um Acylgruppen, wie beispielsweise um Formyl-, Acetyl-, Propionyl- und Benzoylreste, oder um Kohlensäureestergruppen, beispielsweise Methoxycarbonyl- oder Ethoxycarbonylreste, oder um Kohlensäurethiolestergruppen, wie Methylthiocarbonyl- oder Ethylthiocarbonylreste, oder um Urethangruppen, wie Ethylaminocarbonyl- oder unsubstituierte oder substituierte Phenylaminocarbonylreste. Der substituierte Phenylaminocarbonylrest ist vorzugsweise mit einem C₁- bis C₆-Perfluoralkylrest substituiert.

**R**_{**2**} steht vorzugsweise für ein Wasserstoffatom, eine Methyl- oder eine Acetylgruppe.

**R**_{**3**} steht insbesondere für ein Perfluoralkyl mit n = 1, 2 oder 3. Somit kann **R**_{**3**} für 1,1,1-Trifluormethyl, 1,1,2,2,2-Pentafluorethyl oder 1,1,2,2,3,3,3-Heptafluorpropyl stehen. Außerdem kann **R**_{**3**} für 1,1,1-Trifluorethyloxymethyl stehen, wenn **R**_{**3**} durch die allgemeine Formel CH₂O(CH₂)ₘCₙF₂ₙ₊₁ gegeben ist und in diesem Falle m = 1 und n = 1 sind.

Die erfindungsgemäßen Verbindungen sind insbesondere die folgenden 17α-Fluoralkyl-11β-benzaldoxim-Steroide:
1) 4-[17β-Hydroxy-17α-(1,1,1-trifluormethyl)-3-oxoestra-4,9-dien-11β-yl]benzaldehyd-1 (E)-oxim
2) 4-[17β-Hydroxy-17α-(1,1,1-trifluormethyl)-3-oxoestra-4,9-dien-11β-yl]benzaldehyd-1 (Z)-oxim
3) 4-[17β-Hydroxy-17α-(1,1,1-trifluormethyl)-3-oxoestra-4,9-dien-11β-yl]benzaldehyd-1 (E)-[O-(ethylamino)carbonyl]oxim
4) 4-[17β-Hydroxy-17α-(1,1,2,2,2-pentafluorethyl)-3-oxoestra-4,9-dien-11β-yl]-benzaldehyd-1 (E)-oxim
5) 4-[17β-Hydroxy-17α-(1,1,2,2,2-pentafluorethyl)-3-oxoestra-4,9-dien-11β-yl]-benzaldehyd-1 (E)-O-acetyloxim
6) 4-[17β-Hydroxy-17α-(1,1,2,2,2-pentafluorethyl)-3-oxoestra-4,9-dien-11β-yl]benzaldehyd-1(E)-[O-(ethylamino)carbonyl]oxim
7) 4-[17β-Hydroxy-17α-(1,1,2,2,2-pentafluorethyl)-3-oxoestra-4,9-dien-11β-yl]benzaldehyd-1 (E)-[O-(ethylthio)carbonyl]oxim
8) 4-[17β-Hydroxy-17α-(1,1,2,2,2-pentafluorethyl)-3-oxoestra-4,9-dien-11β-yl]benzaldehyd-1 (E)-[O-(ethoxy)carbonyl]oxim
9) 4-[17β-Hydroxy-17α-(1,1,2,2,2-pentafluorethyl)-3-oxoestra-4,9-dien-11β-yl]benzaldehyd-1 (E)-[O-(methoxy)carbonyl]oxim
10) 4-[17β-Hydroxy-17α-(1,1,2,2,3,3,3-heptafluorpropyl)-3-oxoestra-4,9-dien-11 β-yl]benzaldehyd-1 (E)-oxim
11) 4-[17β-Methoxy-17α-(1,1,1-trifluormethyl)-3-oxoestra-4,9-dien-11β-yl]-benzaldehyd-1 (E)-oxim
12) 4-[17β-Methoxy-17α-(1,1,1-trifluormethyl)-3-oxoestra-4,9-dien-11β-yl]-benzaldehyd-1(E)-[O-(ethylamino)carbonyl]oxim
13) 4-[17β-Methoxy-17α-(1,1,2,2,2-pentafluorethyl)-3-oxoestra-4,9-dien-11β-yl]benzaldehyd-1 (E)-oxim
14) 4-[17β-Methoxy-17α-(1,1,2,2,2-pentafluorethyl)-3-oxoestra-4,9-dien-11β-yl]benzaldehyd-1(E)-O-acetyloxim
15) 4-[17β-Methoxy-17α-(1,1,2,2,2-pentafluorethyl)-3-oxoestra-4,9-dien-11β-yl]benzaldehyd-1 (E)-[O-(ethylamino)carbonyl]oxim
16) 4-[17β-Methoxy-17α-(1,1,2,2,2-pentafluorethyl)-3-oxoestra-4,9-dien-11β-yl]benzaldehyd-1 (E)-{O-[(4'-trifluormethyloxy)phenylamino]carbonyl}oxim
17) 4-[17β-Methoxy-17α-(1,1,2,2,2-pentafluorethyl)-3-oxoestra-4,9-dien-11β-yl]-benzaldehyd-1 (E)-[O-(ethoxy)carbonyl]oxim
18) 4-[17β-Methoxy-17α-(1,1,2,2,2-pentafluorethyl)-3-oxoestra-4,9-dien-11β-yl]-benzaldehyd-1 (E)-[O-(methoxy)carbonyl]oxim
19) 4-[17β-Methoxy-17α-(1,1,2,2,2-pentafluorethyl)-3-oxoestra-4,9-dien-11β-yl]-benzaldehyd-1 (E)-[O-(ethylthio)carbonyl]oxim
20) 4-[17β-Acetoxy-17α-(1,1,2,2,2-pentafluorethyl)-3-oxoestra-4,9-dien-11β-yl]-benzaldehyd-1 (E)-[O-(ethylamino)carbonyl]oxim
21) 4-[17β-Acetoxy-17α-(1,1,2,2,2-pentafluorethyl)-3-oxoestra-4,9-dien-11β-yl]-benzaldehyd-1 (E)-oxim
22) 4-[17β-Hydroxy-17α-[(1,1,1-trifluorethyloxy)methyl]-3-oxoestra-4,9-dien-11β-yl]benzaldehyd-1(E)-oxim

In den Dokumenten zum Stand der Technik werden die erfindungsgemäßen 17α-Fluoralkyl-11β-benzaldoxim-Steroide und deren Ester-, Thiolester- oder Urethan-Derivate nicht offenbart. Die erfindungsgemäßen Verbindungen sind daher neu und aus der Literatur bisher nicht bekannt. Das biologische Wirkprofil der genannten Verbindungen ist nicht beschrieben Es ist nicht möglich, mit einem "Antigestagen" alle potentiellen Anwendungsmöglichkeiten abzudecken (W.Elger, K.Chwalisz, *Reproduktionsmedizin*, 1999, 15, 318-335; I.M.Spitz, H.J. Bennink: *Steroids*, 2000, 65, 837-838). Die erfindungsgemäßen Verbindungen sind zur postmenopausalen Substitutionstherapie, auch in Kombination mit einem Estrogen, geeignet oder können zur Behandlung von gynäkologischen Erkrankungen, wie Uterusmyomen oder dysmenorischen Beschwerden eingesetzt werden.

Die vorliegende Erfindung betrifft auch ein Herstellverfahren für die erfindungsgemäßen 17α-Fluoralkyl-11β-benzaldoxim-Steroide.

Die 17α-Fluoralkylgruppen können in das Steroidgerüst eingeführt werden, indem nach an sich bekannten Methoden von einem 17-Keton ausgegangen wird. So ist von Ruppert die Herstellung des Trifluormethyltrimethylsilans beschrieben worden *(Tetrahedron Letters*, 1984, 25, 2195), das in Gegenwart von Tetrabutylammoniumfluorid zur Einführung der Trifluormethylgruppe aus Aldehyden und Ketonen bestens geeignet ist. (R. Krishnamurti, D.R. Bellew, G.K.S. Prakash, *J*. *Org. Chem.,* 1991, 56, 984 und Lamberth, *J. prakt. Chem.,* 1996, 338, 586-587, Ruppert's Reagent).

Zur Einführung der homologen Fluoralkylgruppen in das Steroidgerüst kann von 17-Ketonen ausgegangen werden. Hierzu sind Verfahren beschrieben worden, bei denen Fluoralkylverbindungen mit der allgemeinen Formel Halogen-CₙF₂ₙ₊₁ mit Metallen zu metallorganischen Verbindungen der Alkali- oder Erdalkalireihe mit der allgemeinen Formel Metall-CₙF₂ₙ₊₁ in situ umgesetzt werden. Letztere können anschließend mit den 17-Ketonen zu 17α-Fluoralkyl-17β-hydroxyverbindungen umgesetzt werden. (DE 197 06 061 A1). Die 17α-Fluoralkoxymethylgruppierung kann vorzugsweise durch Ringöffnungreaktion aus einem entsprechenden 17(20)-Spiroepoxid eingeführt werden [Ponsold, Hübner, Schnabel, Strecke, *Arzneimittel-Forschung (Drug Res.),* 24 (1974) 896-900] eingeführt werden.

Das Verfahren zur Herstellung der Ausgangsmaterialien mit der allgemeinen Formel **II:** 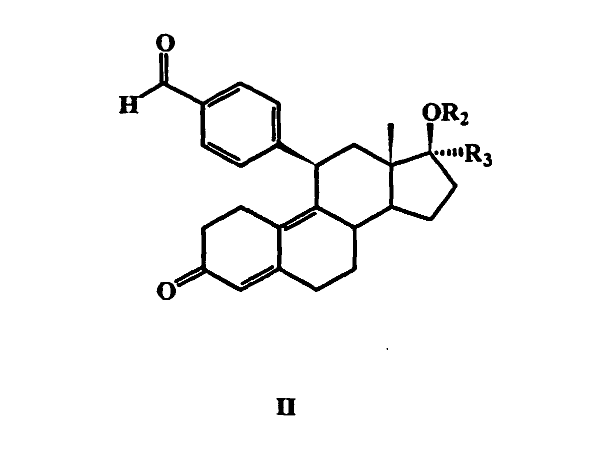 die zur Herstellung der erfindungsgemäßen Verbindungen mit der allgemeinen Formel **I** benötigt werden, ist in EP 0 411 733 A2 und DE 43 32 283 A1 beschrieben:
Zur Herstellung der Verbindungen mit der allgemeinen Formel **II** kann beispielsweise eine mit der nachfolgenden allgemeinen Formel **III** angegebene Verbindung verwendet werden, wobei R^{3'}, R^{4'}, R^{6'}, R^{7'} und R^{8'} die Bedeutung der in EP 0 411 733 A2 in Formel **II** angegebenen entsprechenden Reste R^{3'}, R^{4'}, R⁶, R⁷ bzw. R⁸ haben: 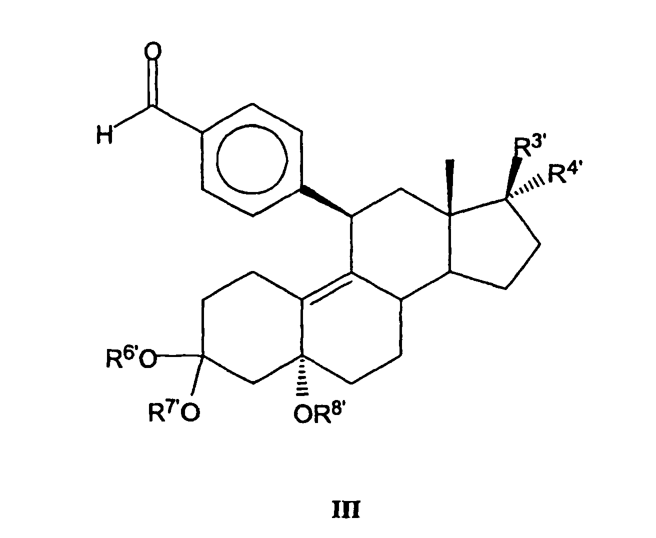 und diese hierzu unter Säurebehandlung in einem mit Wasser mischbaren Lösungsmittel, gegebenenfalls unter Erwärmung, in eine Verbindung überführt werden, bei der die Reste in 3-Stellung zu einer 3-Oxo-Gruppe umgesetzt und durch Austritt der Hydroxygruppe in 5α-Stellung eine Δ^{4,5}-Doppelbindung im Steroidgrundgerüst gebildet werden. Hierzu sind in EP 0 411 733 A2 nähere Angaben enthalten, die hiermit als Offenbarung in die vorliegende Anmeldung aufgenommen werden. Die zur Herstellung der Verbindungen mit der allgemeinen Formel **III** eingesetzten Verfahren, die von den letztendlich gewünschten Substituenten der erfindungsgemäßen Verbindungen abhängen, sind in EP 0 411 733 A2 ebenfalls näher angegeben. Daher wird die sich darauf beziehende Offenbarung in diesem Dokument ebenfalls in die vorliegende Anmeldung aufgenommen. Die entsprechenden Angaben in DE 43 32 283 A1 zur Herstellung der Verbindungen mit der allgemeinen Formel **II** werden ebenfalls als Offenbarung in die vorliegende Anmeldung mit aufgenommen.

Zur Bildung der Benzaldoximgruppe und damit zur Herstellung der erfindungsgemäßen Verbindungen mit der allgemeinen Formel **I** wird in erfindungsgemäßer Weise vorgeschlagen, die durch Einführung der fluorierten Alkylgruppe in 17β-Stellung erhaltene Verbindung mit der allgemeinen Formel **II** mit einem Salz eines Hydroxylamins in einem basischen Lösungsmittel umzusetzen, so dass ein 17α-Fluoralkyl-11β-benzaldoxim-Steroid entsteht, worin **R**_{**1**} Wasserstoff ist, und diese Verbindung anschließend gegebenenfalls zu verestern, zu verethem oder in ein entsprechendes Carbamat, Kohlensäure- oder Thiokohlensäure-Derivat zu überführen. Die weiteren Reste **R**_{**2**} und **R**_{**3**} in dieser allgemeinen Formel haben die weiter oben angegebenen Bedeutungen. Das Salz des Hydroxylamins ist dabei vorzugsweise ein Hydrochlorid oder Hydrosulfat. Das basische Lösungsmittel ist vorzugsweise Pyridin.

Zur Herstellung der erfindungsgemäßen Verbindungen kann die Verbindung mit der allgemeinen Formel **II** in einer alternativen Ausführungsform der Erfindung auch mit einer Verbindung mit der allgemeinen Formel NH₂―O―R₁, worin **R**_{**1**} die vorgenannte Bedeutung hat, umgesetzt werden. Auch diesbezüglich wird auf die entsprechende Beschreibung in DE 43 32 283 A1 verwiesen, die hiermit in die Offenbarung der vorliegenden Anmeldung mit aufgenommen wird.

Die Methoden für die *in vitro-* und die *in vivo*-Tests mit den erfindungsgemäßen Verbindungen können EP 0 411 733 A2 sowie DE 43 32 283 A1 entnommen werden:

Die erfindungsgemäßen 17α-Fluoralkyl-11β-benzaldoxim-Steroide werden am Progesteronrezeptor gebunden (vgl. **Tab.1**) und besitzen im Vergleich zu RU 486 (11β-(4-Dimethylaminophenyl)-17β-hydroxy-17α-propinyl-estra-4,9-dien-3-on) in der Regel eine deutlich reduzierte antiglucocorticoide Wirkung, nachgewiesen durch die verminderte Glucocorticoid-Rezeptorbindung *in vitro* (vgl. **Tab.1**).

**Tabelle 1**

| Rezeptorbindung von 17α-Fluoralkyl-11β-benzaldoxim-Steroiden | | |
|---|---|---|
| Verbindung nach Beispiel | relative molare Bindungsaffinität RBA (%) zum Progesteronrezeptor Progesteron = 100 % | relative molare Bindungsaffinität RBA (%) zum Glucocorticoidrezeptor Dexamethason = 100 % |
| | | |
| 1 | 272 | 163 |
| 2 | 123 | 113 |
| 3 | 98 | 187 |
| 4 | 23 | 10 |
| 5 | 0,5 | 10 |
| 6 | 230 | 84 |
| zum Vergleich: | | |
| RU 486 (Mifepriston)* | 506 | 685 |
| Onapriston | 22 | 39 |

| | | |
|---|---|---|
| * RU 486: 11β-(4-Dimethylaminophenyl)-17β-hydroxy-17α-propinyl-estra-4,9-dien-3-on | | |

**Tabelle 2**

| Frühabortive Wirkung bei der Ratte nach subcutaner Applikation vom 5.-7.-Graviditätstag [Applikation 0,2 ml /Tier/Tag in Benzoylbenzoat/Rizinusöl ( 1+4 v/v) | | | |
|---|---|---|---|
| Substanz | Dosis (mg/Tier/Tag) | Komplette Graviditätshemmung* | |
| | | N^{#}/N | |
| Vehikel | | 0/6 | 0 |
| Beispiel 1 | 1 | 4/4 | 100 |
| | 0,3 | 4/4 | 100 |
| | 0,1 | 1/4 | 75 |
| | 0,03 | 0/6 | 0 |
| Beispiel 2 | 1 | 5/5 | 100 |
| Beispiel 3 | 1 | 4/4 | 100 |
| Beispiel 4 | 1 | 2/4 | 50 |
| Beispiel 6 | 1 | 4/4 | 100 |
| | 0,3 | 4/4 | 100 |
| | 0,1 | 0/4 | 0 |
| RU 486** | 3 | 5/5 | 100 |
| | 1 | 1/5 | 20 |
| | 0,3 | 0/5 | 0 |
| ZK 230 211*** | 3 | 4/4 | 100 |
| | 1 | 3/4 | 75 |
| | 0,3 | 0/6 | 0 |

| | | | |
|---|---|---|---|
| * leere Uteri | | | |
| ** RU 486: 11β-(4-Dimethylaminophenyl)-17β-hydroxy-17α-propinyl-estra-4,9-dien-3-on | | | |
| *** ZK 230211: 11β-(4-Acetylphenyl)-17β-hydroxy-17α-(1,1,2,2,2-pentafluorethyl)-estra-4,9-dien-3-on N: Zahl der angepaarten Weibchen N^{#}: Zahl der nichtgraviden Weibchen | | | |

Die erfindungsgemäßen Verbindungen sind zur postmenopausalen Substitutionstherapie und zur Behandlung von gynäkologischen Erkrankungen, wie Uterusmyomen, sowie zur Behandlung dysmenorischer Beschwerden geeignet.

Gegenstand der vorliegenden Erfindung sind auch Arzneistoffe (pharmazeutische Präparate) zur oralen, rektalen, subcutanen, intravenösen oder intramuskulären Anwendung, die zusammen mit üblichen Trägem und gegebenenfalls Verdünnungsmitteln mindestens eine Verbindung mit der allgemeinen Formel **I** als Wirkstoff enthalten.

Erfindungsgemäße Arzneimittel werden mit den üblichen festen oder flüssigen Trägerstoffen und/oder Verdünnungsmitteln und den üblicherweise allgemein eingesetzten Hilfsstoffen entsprechend der gewünschten Applikationsart in einer geeigneten Dosierung und in an sich bekannter Weise hergestellt. Bei einer bevorzugten oralen Darreichungsform werden vorzugsweise Tabletten, Filmtabletten, Dragees, Kapseln, Pillen, Pulver, Lösungen oder Suspensionen auch als Depotform zubereitet.

Daneben sind parenterale Arzneiformen, wie Injektionslösungen oder aber Suppositorien in Betracht zu ziehen.

Arzneiformen als Tabletten können beispielsweise durch Mischen des Wirkstoffes mit bekannten Hilfsstoffen, wie Dextrose, Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Magnesiumstearat oder Talk, und/oder Mitteln, die einen Depoteffekt erzielen können, wie Carboxypolymethylen, Carboxymethylcellulose, Celluloseacetatphthalat oder Polyvinylacetat, erhalten werden. Die Tabletten können auch aus mehreren Schichten bestehen.

Analog lassen sich Dragees durch Überziehen von analog zu den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker bereiten. Die Drageehülle kann dabei auch aus mehreren Schichten bestehen, wobei beispielsweise die oben genannten Hilfsstoffe verwendet werden.

Die Lösungen oder Suspensionen mit dem erfindungsgemäßen Wirkstoff können zur Verbesserung des Geschmacks mit Stoffen, wie Saccharin, Cyclamat oder Zucker, und/oder mit Aromastoffen, wie Vanillin oder Orangeextrakt, versetzt werden. Weiterhin können sie mit Suspendierhilfsstoffen, wie Natriumcarboxymethylcellulose oder Konservierungsmitteln, wie p-Hydroxybenzoesäure, vermischt werden.

Die Kapseln können durch Mischen des Arzneistoffes mit Trägem, wie Milchzucker oder Sorbit, hergestellt werden, die dann in die Kapseln eingebracht werden.

Suppositorien können vorzugsweise durch Mischen des Wirkstoffes mit geeigneten Trägermaterialien, wie Neutralfetten oder Polyethylenglykolen oder dessen Derivaten, hergestellt werden.

Die galenische Zubereitung enthält die Wirkstoffe in einer Menge von 1 - 100 mg, wobei bei einer Anwendung am Menschen Mengen im Bereich von 1 - 600 mg pro Tag benötigt werden.

Durch die nachfolgenden Beispiele wird die vorliegende Erfindung erläutert, aber nicht eingeschränkt.

### Beispiel 1:

### a) Herstellung der Ausgangsverbindung:

### 4-[17β-Hydroxy-17α-(1,1,1-trifluormethyl)-3-oxoestra-4,9-dien-11β-yl]benzaldehyd

1g 3,3-Dimethoxy-11β-{[4-(1,1-ethylendioxy)methyl]phenyl}-5α-hydroxy-estr-9-en-17-on wird in 30 ml abs. THF gelöst, mit 1,0 g Molsieb 3Å versetzt und 30 Minuten lang unter Argon gerührt. Man kühlt auf 0°C ab, tropft 1,5 ml Trifluormethyltrimethylsilan zu, rührt 10 Minuten lang nach und gibt dann 1 g Tetramethylammoniumfluorid zu. Nach 10 min bei 5°C wird die Reaktionslösung durch Zugabe von 10 ml 1n HCl zersetzt. Man lässt auf Raumtemperatur kommen, gibt jeweils 100 ml Wasser und Essigester zu, trennt die Phasen, wäscht die organische Phase neutral, trocknet über Natriumsulfat, filtriert die organische Phase ab und engt unter Vakuum ein. Nach Zugabe von Aceton verbleiben 1,05 g gelbe Kristalle. Umkristallisation aus Aceton und Behandlung mit *tert*-Butylmethylether ergeben 480 mg 4-[17β-Hydroxy-17α-(1,1,1-trifluormethyl)-3-oxoestra-4,9-dien-11β-yl]benzaldehyd. Die Mutterlauge wird mittels Chromatographie gereinigt und ergibt weitere 320 mg Aldehydprodukt.
Schmp.: 284 - 292°C (Aceton)
α_{D} = + 221° (CHCl₃)
¹H-NMR: [300 MHz, CDCl₃, TMS]: 0,58 (s, 3H, H-18); 4,51 (d, 1H, J = 7,1 Hz, H-11α), 5,81 (s, 1 H, H-4), 7,38 (d, 2H, J = 8,3 Hz, CH-arom.); 7,81 (d, 2H, J = 8,3 Hz), 9,97 (s, 1 H, CH=O).
MS (m/e, 70 eV): 444,19061 (M⁺, 100 %), 426.18390 (M⁺ -H₂O).

### b) Herstellung der erfindungsgemäßen Verbindung:

### 4-[17β-Hydroxy-17α-(trifluormethyl)-3-oxoestra-4,9-dien-11β-yl]benzaldehyd-(1E)-oxim (Verbindung Nr. 1):

549 mg 4-[17β-Hydroxy-17α-(trifluormethyl)-3-oxoestra-4,9-dien-11β-yl]benzaldehyd werden in 5 ml Pyridin gelöst, mit 83 mg Hydroxylaminhydrochlorid versetzt und 1,5 Stunden lang bei Raumtemperatur gerührt. Danach wird die Lösung in Eiswasser eingerührt. Der Niederschlag wird abgesaugt, mit Wasser gewaschen und getrocknet. Das Rohprodukt wird mittels Chromatographie an Kieselgel (0,05-0,063 mm) mit einem Hexan/Essigester-Gradienten gereinigt. Es werden 493 mg Rohprodukt erhalten, die aus tert-Butylmethylether/n-Hexan umkristallisiert werden.

Schmp.: 163 - 167°C unter Zersetzung (*tert*-Butylmethylether/*n*-Hexan)
α_{D} = + 244° (CHCl₃)
¹H-NMR: [300 MHz, CDCl_{3,} TMS]: 0,60 (s, 3H, H-18); 4,44 (d, 1H, J = 7,1 Hz, H-11α), 5,80 (s, 1H, H-4), 7,20 (d, 2H, J = 8,3 Hz, CH-arom.); 7,50 (d, 2H, J = 8,3 Hz); 7,70 (s, 1 H, NOH), 8,10 (s, 1 H CH=N).
MS (m/e, 70 eV): 459,20001 (M⁺), 442,199931 (M⁺ - OH, 100 %).

### Beispiel 2:

### a) Herstellung der Ausgangsverbindung:

### 4-[17β-Hydroxy-17α-(1,1,2,2,2-pentafluorethyl)-3-oxoestra-4,9-dien-11β-yl]-benzaldehyd

20 g 3,3-Dimethoxy-11β-{[4-(1,1-ethylendioxy)methyl]phenyl}-5α-hydroxy-estr-9-en-17-on werden in 600 ml Diethylether suspendiert und unter Rühren auf - 78°C gekühlt. Es werden 48 g Pentafluorethyliodid zugegeben und anschließend langsam 76 ml einer 1,5 ml Lösung von Methyllithium-Lithiumbromidkomplex in Diethylether zugetropft. Es wird 2 Stunden lang bei -78°C gerührt und dann auf 2 l gesättigte Natriumhydrogencarbonatlösung gegossen. Anschließend wird mit Essigsäureethylester extrahiert, getrocknet und eingeengt. Der Rückstand wird in 200 ml 70%iger Essigsäure aufgenommen und 60 min lang auf 60°C erwärmt. Man lässt abkühlen und versetzt mit 400 ml Wasser, wobei das Produkt ausfällt. Der Niederschlag wird abgesaugt, mit Wasser gewaschen und mit *tert*-Butylmethylether ausgekocht. Man erhält 4-[17β-Hydroxy-17α-(1,1,2,2,2-pentafluorethyl)-3-oxoestra-4,9-dien-11β-yl]benzaldehyd.
Schmp.: 220 - 230°C (*tert*-Butylmethylether)
¹H-NMR (CDCl₃) : 0,58 (s, 3H, H-18), 4,52 (d, 1H, J = 7,03 Hz, H-11α), 5,81 (s, 1H, H-4), 7,38 (d, 2H, J = 7,81 Hz, CH-arom.); 7,81 (d, 2H, J = 8,6 Hz), 9,96 (s, 1 H, CH=O).
¹⁹F-NMR : 77,8 (3F,CF₃), 119 (2F,CF₂)

### b) Herstellung der erfindungsgemäßen Verbindung:

### 4-[17β-Hydroxy-17α-(1,1,2,2,2-pentafluorethyl)-3-oxoestra-4,9-dien-11β-yl]-benzaldehyd-(1E)-oxim (Verbindung Nr. 4)

2,5 g 4-[17β-Hydroxy-17α-(1,1,2,2,2-pentafluorethyl)-3-oxoestra-4,9-dien-11β-yl]-benzaldehyd werden in 32 ml Pyridin gelöst und mit 450 mg HydroxylaminHydrochlorid innerhalb von 4 Stunden bei Raumtemperatur umgesetzt. Man gießt in Eiswasser ein, saugt den Niederschlag ab, trocknet und reinigt mittels Chromatographie. Nach Umkristallisation aus Aceton wird 4-[17β-Hydroxy-17α-(1,1,2,2,2-pentafluorethyl)-3-oxoestra-4,9-dien-11β-yl]benzaldehyd-(1E)-oxim erhalten.
Schmp.: 220 - 230°C
¹H-NMR: 0,59 (s, 3H, H-18); 4,45 (d, 1 H, J = 6,6 Hz, H-11α), 5,80 (s, 1 H, H-4), 7,20 (d, 2H, J = 8,2 Hz, CH-arom.); 7,48 (d, 2H, J = 8,2 Hz); 8,10 (s, 1H CH=N) 8,24 (s, 1H, NOH),
¹⁹F-NMR : 77,3 (3F,CF₃), 119 (2F,CF₂)

### Beispiel 3:

### 4-[17β-Hydroxy-17α-(1,1,2,2,2-pentafluorethyl)-3-oxoestra-4,9-dien-11β-yl]-benzaldehyd-1-on-(1E)-{O-(ethylamino)carbonyl-oxim (Verbindung Nr. 6)

1,4 g 4-[17β-Hydroxy-17α-(1,1,2,2,2-pentafluorethyl)-3-oxoestra-4,9-dien-11β-yl]-benzaldehyd-(1 E)-oxim (Verbindung Nr. 4) werden in 50 ml Toluol gelöst, mit 2,26 ml Triethylamin sowie 1,2 ml Ethylisocyanat versetzt und auf 60°C erwärmt. Das Gemisch wird 1,5 Stunden lang gerührt, auf 10°C gekühlt, mit 25 ml wässriger Ammoniaklösung sowie 100 ml Essigsäureethylester versetzt und 30 min lang nachgerührt. Nach der Phasentrennung wird die organische Phase mit Wasser neutral gewaschen, mit Natriumsulfat getrocknet und unter Vakuum eingeengt. Der Rückstand wird aus Essigsäureethylester umkristallisiert. Man erhält 4-[17β-Hydroxy-17α-(1,1,2,2,2-pentafluorethyl)-3-oxoestra-4,9-dien-11β-yl]benzaldehyd-1-on-(1E)-{O-(ethylamino)carbonyl-oxim.
Schmp.: 140 - 146°C (Essigsäureethylester)
¹H-NMR : 0,59 (s, 3H, H-18), 1,24 (t, 3H J= 7,2 Hz, CH₂ CH₃) 4,12 (d, J = 6,8 Hz, 1H, H-11), 5,80 (s, 1H, H-4), 6,2(t, 1 H J= 5,7 Hz, -NH-) 7,27 und 7,59 (2d , je 2H, J=9 Hz, CH-arom.), 8,29 (s, 1H, CH=N).
¹⁹F-NMR(386 MHz): 77,6 (3F,CF₃), 119 (2F,CF₂)

### Beispiel 4:

### a) Herstellung der Ausgangsverbindung:

### a1) Stufe A:

### 11β-{[4-(1,1-Ethylendioxy)methyl]phenyl}-17α-(1,1,2,2,2-pentafluorethyl)-3,3,17β-trimethoxy-estr-9-en-5α-ol

602 mg 3,3-Dimethoxy-11β-{[4-(1,1-ethylendioxy)-methyl]phenyl}-17α-(1,1,2,2,2-pentafluorethyl)-estr-9-en-5α,17β-diol werden unter Kühlung und Argon-Schutzgas in 5 ml Toluol gelöst. 232 mg Kalium-*tert*-butanolat werden portionsweise im Wechsel mit Methyliodid in Toluol über 2 Stunden zugefügt. Nach 3 Stunden wird mit 20 ml Wasser versetzt, und die Phasen werden getrennt. Die wässrige Phase wird mit Toluol nachextrahiert, der vereinigte Extrakt neutral gewaschen und über Natriumsulfat getrocknet.

Nach Einengung werden 662 mg Rohprodukt als Schaum erhalten, das ohne weitere Reinigung in die Stufe B eingesetzt wird.
¹H-NMR: [400 MHz, CDCl_{3,} TMS]: 0,56 (s, 3H, H-18); 2,35 (s, 1H, OH), 3,20 und 3,22 (2s, je 3 H, 2 x OCH₃), 3,33 (s, 3H, OCH₃), 4,08 (m, 4H, Ethylenketal), 4,29 (d, 1H, J = 7,2 Hz, H-11α), 4,62 (s, 1H, CH), 5,74 (s, 1H, 5-OH), 7,23 (d, 2H, J = 8,0 Hz, CH-arom.); 7,37 (d, 2H, J= 8,0 Hz, arom. CH)

### a2) Stufe B:

### 4-[17β-Methoxy-17α-(1,1,2,2,2-pentafluorethyl)-3-oxoestra-4,9-dien-11β-yl]-benzaldehyd

640 mg 11β-{[4-(1,1-Ethylendioxy)methyl]phenyl}-17α-(1,1,2,2,2-pentafluorethyl)-3,3,17β-trimethoxy-estr-9-en-5α-ol werden unter Argon-Schutzgas in 10 ml Aceton gelöst, mit 300 mg *p*-Toluolsulfonsäure versetzt und 1 Stunde lang bei Raumtemperatur gerührt. Die Reaktionslösung wird in 500 ml Eiswasser eingerührt, wobei ein Produkt flockig ausfällt. Mit wässriger Natriumhydrogencarbonat-Lösung wird neutralisiert, der Niederschlag abgesaugt, das Filtrat mit Wasser gewaschen und getrocknet. Man erhält 411 mg Rohprodukt, das mit Hilfe der präparativen Schichtchromatographie gereinigt wird.
Schmp.: 160 - 162°C (CH₂Cl₂, *tert*-Butylmethylether, n-Hexan)
α_{D} = + 200° (CHCl₃)
¹H-NMR: [400 MHz, CDCl₃, TMS]: 0,61 (s, 3H, H-18); 3,36 (s, 3H, OCH₃), 4,49 (d, 1H, J = 6,8 Hz, H-11α), 5,80 (s, 1 H, H-4), 7,38 (d, 2H, J = 7,6 Hz, CH-arom.); 7,81 (d, 2H, J = 8,8 Hz), 9,96 (s, 1 H, CH=O).
¹⁹F-NMR (386 MHz): 78,3 (s), 110,5 (d) und 113,7 (d)

### b) Herstellung der erfindungsgemäßen Verbindung:

### 4-[17β-Methoxy-17α-(1,1,2,2,2-pentafluorethyl)-3-oxoestra-4,9-dien-11β-yl]-benzaldehyd-(1E)-oxim (Verbindung Nr. 13)

Diese Verbindung wird entsprechend der Vorschrift gemäß Beispiel 2 aus 4-[17β-Methoxy-17α-(1,1,2,2,2-pentafluorethyl)-3-oxoestra-4,9-dien-11β-yl]-benzaldehyd mit Hydroxylaminhydrochlorid in Pyridin hergestellt.
Schmp.: 122 - 124°C (*tert*-Butylmethylether)
α_{D} = + 188° (CHCl₃)
¹H-NMR: [400 MHz, CDCl_{3,} TMS]: 0,64 (s, 3H, H-18); 3,36 (s, 3H, OCH₃), 4,44 (d, 1 H, J = 6,4 Hz, H-11α), 5,79 (s, 1H, H-4), 7,21 (d, 2H, J = 7,6 Hz, CH-arom.); 7,49 (d, 2H, J = 8,4 Hz); 8,02 (s, 1H, NOH), 8,10 (s, 1 H CH=N).

### Beispiel 5:

### 4-[17β-Methoxy-17α-(1,1,2,2,2-pentafluorethyl)-3-oxoestra-4,9-dien-11β-yl]-benzaldehyd-1-on-(1E)-{O-[(4'-trifluormethoxy)phenylamino]carbonyl-oxim

Diese Verbindung wird entsprechend Beispiel 3 aus 4-[17β-Methoxy-17α-(1,1,2,2,2-pentafluorethyl)-3-oxoestra-4,9-dien-11β-yl]benzaldehyd-(1E)-oxim mit 4-Trifluormethoxymethylphenylisocyanat in Toluol hergestellt.
farbloser Schaum
α_{D} = + 149° (Chloroform)
¹H-NMR (400 MHz, CDCl₃, δ in ppm, TMS): 0,64 (s, 3H, H-18), 3,36 (s, 3H, OCH₃), 4,48 (d, J = 6,8 Hz, 1 H, H-11), 5,80 (s, 1H, H-4), 7,21 und 7,54 (2d , je 2H, J=8,8 Hz, CH-arom.), 7,31 und 7,65 (2d, je 2H, J= 8,0 Hz, CH-arom.), 8,16 (s, 1 H, NH), 8,37 (s, 1 H, CH=N).
¹⁹F-NMR(386 MHz): 58,4 (s), 78,3 (s), 110,7(d) und 113,8 (d)

### Beispiel 6:

### a) Herstellung des Ausgangsmaterials:

### a1) Stufe A:

### 4-[3,3-Dimethoxy-5α,17β-dihydroxy-17α-[(1,1,1-trifluorethyloxy)methyl]-estr-9-en-11β-yl]benzaldehyd-ethylenketal

70 ml DMSO werden mit 1,54 g Kalium-*tert*-butanolat bei Raumtemperatur gerührt. Man tropft 0,8 ml Trifluorethanol zu, rührt 10 Minuten lang nach und fügt anschließend eine Lösung von 1,36 g 4-(3,3-Dimethoxy-5α-hydroxy-17(S)-spiroepoxy-estr-9-en-11β-yl)-benzaldehyd-ethylenketal in 30 ml DMSO zu. Unter Argonschutz wird auf 40°C erwärmt. Nach jeweils 3 und 5 Stunden werden weitere 0,8 ml Trifluorethanol und 1,54 g Kalium-*tert*-butanolat zugefügt. Nach 12 Stunden wird mit wässriger NH₄Cl-Lösung versetzt und mit Toluol ausgeschüttelt. Nach der üblichen Aufarbeitung werden 2,8 g Rohprodukt erhalten, das durch Chromatographie an Kieselgel gereinigt wird. Dieses gereinigte Produkt wird direkt in die nächste Stufe eingesetzt.
α_{D} = - 1° (CHCl₃)
MS: m/e 596,29809 M⁺ (C₃₂H₄₃F₃O₇)

### a2) Stufe B:

### 4-[17β-Hydroxy-17α-[(1,1,1-trifluorethyloxy)methyl]-3-oxoestra-4,9-dien-11β-yl]-benzaldehyd

862 mg 4-[3,3-Dimethoxy-5α,17β-dihydroxy-17α-(1,1,1-trifluorethyloxymethyl)-estr-9-en-11β-yl-benzaldehyd-ethylenketal werden in 15 ml Aceton gelöst, mit 1,5 ml Wasser und 350 mg *p*-Toluolsulfonsäure versetzt und 1 Stunde lang bei Raumtemperatur gerührt. Man verdünnt mit Wasser und extrahiert mit Methylenchlorid. Die organische Phase wird neutral gewaschen, getrocknet und eingeengt. Man erhält 663 mg 4-[17β-Hydroxy-17α-(1,1,1-trifluorethyloxymethyl)-3-oxoestra-4,9-dien-11β-yl]benzaldehyd als Schaum. Das Rohprodukt wird durch präparative Schichtchromatographie gereinigt.
Schmp.: 100 - 103°C (Ether)
α_{D} = + 169° (CHCl₃)
¹H-NMR: [400 MHz, CDCl₃, TMS]: 0,53 (s, 3H, H-18); 3,45 und 3,91 (2m, 2 xCH₂); 4,45 (d, 1 H, J = 7,2 Hz, H-11α), 5,81 (s, 1H, H-4), 7,37 (d, 2H, J = 8,1 Hz, CH-arom.); 7,81 (d, 2H, J = 8,1 Hz), 9,98 (s, 1H, CH=O).

### b) Herstellung der erfindungsgemäßen Verbindung:

### 4-[17β-Hydroxy-17α-(1,1,1-trifluorethyloxymethyl)-3-oxoestra-4,9-dien-11β-yl]-benzaldehyd-(1E)-oxim (Verbindung Nr. 22)

Zur Herstellung der Titelverbindung werden 340 mg 4-[17β-Hydroxy-17α-(1,1,1-trifluorethyloxymethyl)-3-oxoestra-4,9-dien-11β-yl]benzaldehyd gemäß Beispiel 2 umgesetzt. Es werden 326 mg Rohprodukt erhalten. Das Rohprodukt wird durch präparative Schichtchromatographie an Kieselgel PF_{254 nm} gereinigt.
Schmelzpunkt: 132 - 136°C (Ether)
α_{D} = + 182°
¹H-NMR (400 MHz, CDCl₃, δ in ppm, TMS): 0,55 (s, 3H, H-18), 3,45 und 3,89 (m, je 2H, CH₂), 4,39 (d, J = 6,8 Hz, 1 H, H-11), 5,80 (s, 1 H, H-4), 7,20 und 7,49 (2d, je 2H, J= 8,4 Hz, CH-arom.), 7,60 (s, 1 H, OH), 8,11 (s, 1H, CH=N). ¹⁹F-NMR(386 MHz): 58,4 (s), 78,3 (s), 110,7(d) und 113,8 (d)

### Beispiel 7:

### a) Herstellung der Ausgangsverbindung:

### 4-[17β-Hydroxy-17α-(1,1,2,2,3,3,3-heptafluorpropyl)-3-oxoestra-4,9-dien-11β-yl] benzaldehyd

965 mg 3,3-Dimethoxy-11β-{[4-(1,1-ethylendioxy)methyl]phenyl}-5α-hydroxy-estr-9-en-17-on wird in 35 ml abs. THF gelöst, mit 1,0 g Molsieb 3Å versetzt und 30 Minuten lang unter Argon gerührt. Man kühlt auf 0°C ab, tropft 0,5 ml 1,1,2,2,3,3,3-Heptafluorpropyltrimethylsilan zu, rührt 10 Minuten lang nach und gibt dann 55 mg Tetrabutylammoniumfluorid zu. Nach 10 Minuten bei 5°C wird die Reaktionslösung durch Zugabe von 10 ml 1n HCl zersetzt. Man lässt auf Raumtemperatur kommen, rührt 2 Stunden lang nach, kühlt wieder auf 0°C ab und fügt weitere 0,5 ml 1,1,2,2,3,3,3-Heptafluorpropyltrimethylsilan zu. Nach 15 Minuten wird die Lösung durch Zugabe von 15 ml 1N HCl hydrolysiert. Nach weiteren 30 Minuten werden 100 ml gesättigte Ammoniumchlorid-Lösung zugefügt, und die Lösung wird mit Essigester extrahiert. Man wäscht die organische Phase neutral, trocknet über Natriumsulfat, filtriert die organische Phase ab und engt unter Vakuum ein. Das braune Rohprodukt (924 mg) wird mittels Chromatographie an Kieselgel mit Essigester/*n*-Hexan 1:2 gereinigt. Umkristallisation aus Aceton/*n*-Hexan ergibt 485 mg 4-[17β-Hydroxy-17α-(1,1,2,2,3,3,3-heptafluorpropyl)-3-oxoestra-4,9-dien-11β-yl]benzaldehyd.

### b) Herstellung der erfindungsgemäßen Verbindung:

### 4-[17β-Hydroxy-17α-(1,1,2,2,3,3,3-heptafluorpropyl)-3-oxoestra-4,9-dien-11β-yl] benzaldehyd-(9E)-oxim (Verbindung Nr. 10)

Die Titelverbindung wird aus 4-[17β-Hydroxy-17α-(1,1,2,2,3,3,3-heptafluorpropyl)-3-oxoestra-4,9-dien-11β-yl]benzaldehyd durch Umsetzung mit Hydroxylaminhydrochlorid in Pyridin gemäß Beispiel 2 hergestellt.
Schmp. 170 - 175°C
¹H-NMR: 0,59 (s, 3H, H-18); 4,45 (d, 1H, J = 6,6 Hz, H-11α), 5,80 (s, 1H, H-4), 7,20 (d, 2H, J = 8,2 Hz, CH-arom.); 7,48 (d, 2H, J = 8,2 Hz); 8,10 (s, 1 H CH=N) 8,24 (s, 1H, NOH)

Wie ein Fachmann leicht erkennen kann, sind beliebige Änderungen und Modifikationen der hier dargestellten bevorzugten Ausführungsformen der Erfindung in den Schutzbereich der anliegenden Patentansprüche einbezogen. Dies schließt auch ein, dass beliebige Kombinationen der Merkmale der vorliegenden Erfindung in den Offenbarungsgehalt einbezogen werden.

## Patentansprüche

1. 17α-Fluoralkyl-11β-benzaldoxim-Steroide mit der allgemeinen Formel **I** 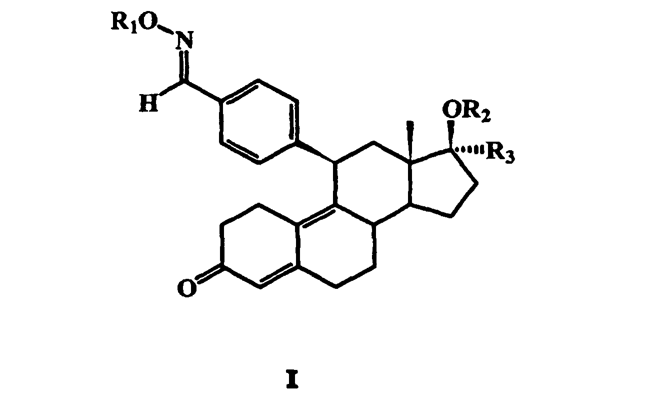 worin
**R**_{**1**} für Wasserstoff, C₁- bis C₆-Alkyl, COR₄, COOR₄, COSR₄ oder CONHR₅ steht, worin **R**_{**4**} C₁- bis C₆-Alkyl oder unsubstituiertes oder substituiertes Aryl ist und worin **R**_{**5**} Wasserstoff, C₁- bis C₆-Alkyl oder unsubstituiertes oder substituiertes Aryl ist,
**R**_{**2**} für Wasserstoff, C₁- bis C₆-Alkyl oder C₁- bis C₆-Acyl steht und
**R**_{**3**} für eine CₙF₂ₙ₊₁-Gruppe, bei der n = 1, 2 oder 3 ist, oder für eine CH₂O(CH₂)ₘCₙF₂ₙ₊₁-Gruppe steht, bei der m = 0 oder 1 und n = 1, 2 oder 3 sind,
sowie deren pharmazeutisch verträgliche Salze.

2. 17α-Fluoralkyl-11β-benzaldoxim-Steroide nach Anspruch 1, **dadurch gekennzeichnet, dass R**_{**1**} für Wasserstoff, Methyl, Formyl, Acetyl, Propionyl, Benzoyl, Methoxycarbonyl, Ethoxycarbonyl, Methylthiocarbonyl, Ethylthiocarbonyl, Ethylaminocarbonyl oder unsubstituiertes oder substituiertes Phenylaminocarbonyl steht.

3. 17α-Fluoralkyl-11β-benzaldoxim-Steroide nach Anspruch 2, **dadurch gekennzeichnet, dass** der substituierte Phenylaminocarbonylrest mit einem C₁- bis C₆-Perfluoralkylrest substituiert ist.

4. 17α-Fluoralkyl-11β-benzaldoxim-Steroide nach einem der Ansprüche 1 - 3, **dadurch gekennzeichnet, dass R**_{**2**} für Wasserstoff, Methyl oder Acetyl steht

5. 17α-Fluoralkyl-11β-benzaldoxim-Steroide nach einem der Ansprüche 1 - 4, **dadurch gekennzeichnet, dass R**₃ für 1,1,1-Trifluormethyl, 1,1,2,2,2-Pentafluorethyl, 1,1,2,2,3,3,3-Heptafluorpropyl oder 1,1,1-Trifluorethyloxymethyl steht.

6. 17α-Fluoralkyl-11β-benzaldoxim-Steroide nach einem der Ansprüche 1 - 5, nämlich
1) 4-[17β-Hydroxy-17α-(1,1,1-trifluormethyl)-3-oxoestra-4,9-dien-11β-yl]-benzaldehyd-1 (E)-oxim
2) 4-[17β-Hydroxy-17α-(1,1,1-trifluormethyl)-3-oxoestra-4,9-dien-11β-yl]-benzaldehyd-1 (Z)-oxim
3) 4-[17β-Hydroxy-17α-(1,1,1-trifluormethyl)-3-oxoestra-4,9-dien-11β-yl]-benzaldehyd-1 (E)-[O-(ethylamino)carbonyl]oxim
4) 4-[17β-Hydroxy-17α-(1,1,2,2,2-pentafluorethyl)-3-oxoestra-4,9-dien-11β-yl]benzaldehyd-1(E)-oxim
5) 4-[17β-Hydroxy-17α-(1,1,2,2,2-pentafluorethyl)-3-oxoestra-4,9-dien-11β-yl]benzaldehyd-1(E)-O-acetyloxim
6) 4-[17β-Hydroxy-17α-(1,1,2,2,2-pentafluorethyl)-3-oxoestra-4,9-dien-11β-yl]benzaldehyd-1(E)-[O-(ethylamino)carbonyl]oxim
7) 4-[17β-Hydroxy-17α-(1,1,2,2,2-pentafluorethyl)-3-oxoestra-4,9-dien-11β-yl]benzaldehyd-1(E)-[O-(ethylthio)carbonyl]oxim
8) 4-[17β-Hydroxy-17α-(1,1,2,2,2-pentafluorethyl)-3-oxoestra-4,9-dien-11β-yl]benzaldehyd-1(E)-[O-(ethoxy)carbonyl]oxim
9) 4-[17β-Hydroxy-17α-(1,1,2,2,2-pentafluorethyl)-3-oxoestra-4,9-dien-11β-yl]benzaldehyd-1(E)-[O-(methoxy)carbonyl]oxim
10) 4-[17β-Hydroxy-17α-(1,1,2,2,3,3,3-heptafluorpropyl)-3-oxoestra-4,9-dien-11 β-yl]benzaldehyd-1(E)-oxim
11) 4-[17β-Methoxy-17α-(1,1,1-trifluormethyl)-3-oxoestra-4,9-dien-11β-yl]-benzaldehyd-1 (E)-oxim
12) 4-[17β-Methoxy-17α-(1,1,1-trifluormethyl)-3-oxoestra-4,9-dien-11β-yl]-benzaldehyd-1 (E)-[O-(ethylamino)carbonyl]oxim
13) 4-[17β-Methoxy-17α-(1,1,2,2,2-pentafluorethyl)-3-oxoestra-4,9-dien-11β-yl]benzaldehyd-1 (E)-oxim
14) 4-[17β-Methoxy-17α-(1,1,2,2,2-pentafluorethyl)-3-oxoestra-4,9-dien-11β-yl]benzaldehyd-1(E)-O-acetyloxim
15) 4-[17β-Methoxy-17α-(1,1,2,2,2-pentafluorethyl)-3-oxoestra-4,9-dien-11β-yl]benzaldehyd-1(E)-[O-(ethylamino)carbonyl]oxim
16) 4-[17β-Methoxy-17α-(1,1,2,2,2-pentafluorethyl)-3-oxoestra-4,9-dien-11β-yl]benzaldehyd-1 (E)-{O-[(4'-trifluormethoxy)phenylamino]carbonyl}oxim
17) 4-[17β-Methoxy-17α-(1,1,2,2,2-pentafluorethyl)-3-oxoestra-4,9-dien-11β-yl]benzaldehyd-1(E)-[O-(ethoxy)carbonyl]oxim
18) 4-[17β-Methoxy-17α-(1,1,2,2,2-pentafluorethyl)-3-oxoestra-4,9-dien-11β-yl]benzaldehyd-1 (E)-[O-(methoxy)carbonyl]oxim
19) 4-[17β-Methoxy-17α-(1,1,2,2,2-pentafluorethyl)-3-oxoestra-4,9-dien-11β-yl]benzaldehyd-1 (E)-[O-(ethylthio)carbonyl]oxim
20) 4-[17β-Acetoxy-17α-(1,1,2,2,2-pentafluorethyl)-3-oxoestra-4,9-dien-11β-yl]benzaldehyd-1(E)-[O-(ethylamino)carbonyl]oxim
21) 4-[17β-Acetoxy-17α-(1,1,2,2,2-pentafluorethyl)-3-oxoestra-4,9-dien-11β-yl]benzaldehyd-1 (E)-oxim
22) 4-[17β-Hydroxy-17α-[(1,1,1-trifluorethyloxy)methyl]-3-oxoestra-4,9-dien-11 β-yl]benzaldehyd-1 (E)-oxim.

7. Verfahren zur Herstellung von 17α-fluoralkylierten 11β-Benzaldoxim-Steroiden mit der allgemeinen Formel **I** 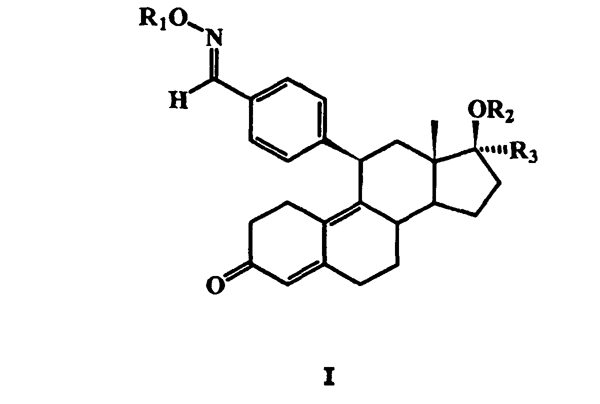 worin die Reste **R**_{**1**}**, R**_{**2**} und **R**_{**3**} die in den Ansprüchen 1 - 5 angegebenen Bedeutungen haben,
**dadurch gekennzeichnet, dass** ein 11 β-Benzaldehyd mit der allgemeinen Formel **II** 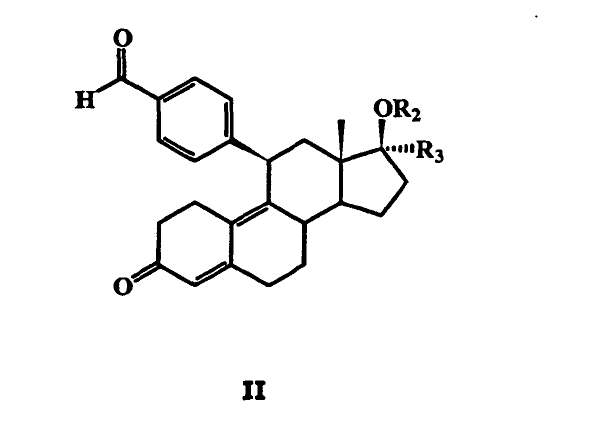 mit einem Salz eines Hydroxylamins in einem basischen Lösungsmittel umgesetzt wird, so dass ein 17α-Fluoralkyl-11β-benzaldoxim-Steroid entsteht, worin **R**_{**1**} Wasserstoff ist, und diese Verbindung gegebenenfalls verestert, verethert oder in ein entsprechendes Carbamat, Kohlensäure- oder Thiokohlensäure-Derivat überführt wird.

8. Verfahren nach Anspruch 7, **dadurch gekennzeichnet, dass** das Salz des Hydroxylamins ein Hydrochlorid oder Hydrosulfat ist.

9. Verfahren nach einem der Ansprüche 7 und 8, **dadurch gekennzeichnet, dass** das basische Lösungsmittel Pyridin ist.

10. Pharmazeutisches Präparat, enthaltend mindestens ein 17α-Fluoralkyl-11β-benzaldoxim-Steroid mit der allgemeinen Formel **I** nach einem der Ansprüche 1 - 6 sowie mindestens einen pharmazeutisch verträglichen Träger.

11. Verwendung der 17α-Fluoralkyl-11β-benzaldoxim-Steroide mit der allgemeinen Formel I nach einem der Ansprüche 1 - 6 zur Herstellung von Arzneimitteln.

## Claims

1. 17α-Fluoroalkyl-11β-benzaldoxime steroids with general formula I 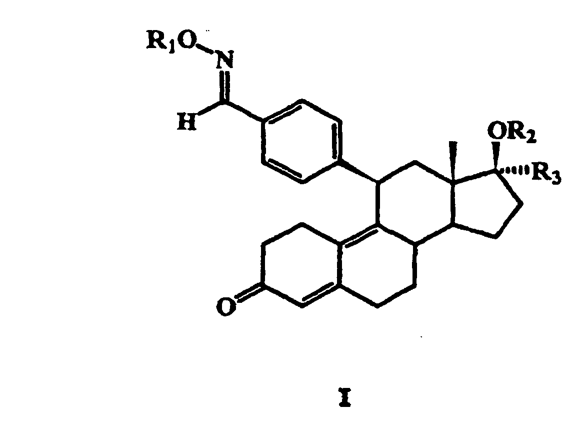 in which
R₁ stands for hydrogen, C₁-C₆ alkyl, COR₄, COOR₄, COSR₄ or CONHR₅, in which R₄ is C₁-C₆ alkyl or unsubstituted or substituted aryl and in which R₅ is hydrogen, C₁-C₆ alkyl or unsubstituted or substituted aryl,
R₂ stands for hydrogen, C₁-C₆ alkyl or C₁-C₆ acyl, and
R₃ stands for a CₙF₂ₙ₊₁ group, in which n = 1, 2 or 3, or a CH₂O(CH₂)ₘCₙF₂ₙ₊₁ group, in which m = 0 or 1 and n = 1, 2 or 3,
as well as their pharmaceutically compatible salts.

2. 17α-fluoroalkyl-11β-benzaldoxime steroids according to claim 1, **characterized in that** R₁ stands for hydrogen, methyl, formyl, acetyl, propionyl, benzoyl, methoxycarbonyl, ethoxycarbonyl, methylthiocarbonyl, ethylthiocarbonyl, ethylaminocarbonyl or unsubstituted or substituted phenylaminocarbonyl.

3. 17α-fluoroalkyl-11β-benzaldoxime steroids according to claim 2, wherein the substituted phenylaminocarbonyl radical is substituted with a C₁-C₆ perfluoroalkyl radical.

4. 17α-fluoroalkyl-11β-benzaldoxime steroids according to one of claims 1 to 3, whereinR₂ stands for hydrogen, methyl or acetyl.

5. 17α-fluoroalkyl-11β-benzaldoxime steroids according to one of claims 1 to 4, wherein R₃ stands for 1,1,1-trifluoromethyl, 1,1,2,2,2-pentafluoroethyl, 1,1,2,2,3,3,3-heptafluoropropyl or 1,1,1-trifluoroethyloxymethyl.

6. 17α-fluoroalkyl-11β-benzaldoxime steroids according to one of claims 1 to 5, namely,
1) 4-[17β-hydroxy-17α-(1,1,1-trifluoromethyl)-3-oxoestra-4,9-dien-11β-yl]benzaldehyde 1 (E)-oxime
2) 4-[17β-hydroxy-17α-(1,1,1-trifluoromethyl)-3-oxoestra-4,9-dien-11β-yl]benzaldehyde 1 (Z)-oxime
3) 4-[17β-hydroxy-17α-(1,1,1-trifluoromethyl)-3-oxoestra-4,9-dien-11β-yl]benzaldehyde 1 (E)-[O-(ethylamino)carbonyl]oxime
4) 4-[17β-hydroxy-17α-(1,1,2,2,2-pentafluoroethyl)-3-oxoestra-4,9-dien-11 β-yl]benzaldehyde 1 (E)-oxime
5) 4-[17β-hydroxy-17α-(1,1,2,2,2-pentafluoroethyl)-3-oxoestra-4,9-dien-11 β-yl]benzaldehyde 1 (E)-O-acetyloxime
6) 4-[17β-hydroxy-17α-(1,1,2,2,2-pentafluoroethyl)-3-oxoestra-4,9-dien-11 β-yl]benzaldehyde 1 (E)-[O-(ethylamino)carbonyl]oxime
7) 4-[17β-hydroxy-17α-(1,1,2,2,2-pentafluoroethyl)-3-oxoestra-4,9-dien-11β-yl]benzaldehyde 1 (E)-[O-(ethylthio)carbonyl]oxime
8) 4-[17β-hydroxy-17α-(1,1,2,2,2-pentafluoroethyl)-3-oxoestra-4,9-dien-11 β-yl]benzaldehyde 1 (E)-[O-(ethoxy)carbonyl]oxime
9) 4-[17β-hydroxy-17α-(1,1,2,2,2-pentafluoroethyl)-3-oxoestra-4,9-dien-11 β-yl)benzaldehyde 1 (E)-[O-(methoxy)carbonyl]oxime
10) 4-[17β-hydroxy-17α-(1,1,2,2,3,3,3-heptafluoropropyl)-3-oxoestra-4,9-dien-11β-yl]benzaldehyde 1(E)-oxime
11) 4-[17β-methoxy-17α-(1,1,1-trifluoromethyl)-3-oxoestra-4,9-dien-11β-yl]benzaldehyde 1(E)-oxime
12) 4-[17β-methoxy-17α-(1,1,1-trifluoromethyl)-3-oxoestra-4,9-dien-11β-yl]benzaldehyde 1 (E)-[O-(ethylamino)carbonyl]oxime
13) 4-[17β-methoxy-17α-(1,1,2,2,2-pentafluoroethyl)-3-oxoestra-4,9-dien-11β-yl]benzaidehyde 1 (E)-oxime
14) 4-[17β-methoxy-17α-(1,1,2,2,2-pentafluoroethyl)-3-oxoestra-4,9-dien-11 β-yl]benzaldehyde 1(E)-O-acetyloxime
15) 4-[17β-methoxy-17α-(1,1,2,2,2-pentafluoroethyl)-3-oxoestra-4,9-dien-11β-yl]benzaldehyde 1 (E)-[O-(ethylamino)carbonyl]oxime
16) 4-[17β-methoxy-17α-(1,1,2,2,2-pentafluoroethyl)-3-oxoestra-4,9-dien-11 β-yl]benzaldehyde 1 (E)-{O-[(4'-trifluoromethoxy)phenylamino)]carbonyl}oxime
17) 4-[17β-methoxy-17α-(1,1,2,2,2-pentafluoroethyl)-3-oxoestra-4,9-dien-11 β-yl]benzaldehyde 1 (E)-[O-(ethoxy)carbonyl]oxime
18) 4-[17β-methoxy-17α-(1,1,2,2,2-pentafluoroethyl)-3-oxoestra-4,9-dien-11β-yl]benzaldehyde 1(E)-[O-(methoxy)carbonyl]oxime
19) 4-[17β-methoxy-17α-(1,1,2,2,2-pentafluoroethyl)-3-oxoestra-4,9-dien-11 β-yl]benzaldehyde 1 (E)-[O-(ethylthio)carbonyl]oxime
20) 4-[17β-acetoxy-17α-(1,1,2,2,2-pentafluoroethyl)-3-oxoestra-4,9-dien-11 β-yl]benzaldehyde 1(E)-[O-(ethylamino)carbonyl]oxime
21) 4-[17β-acetoxy-17α-(1,1,2,2,2-pentafluoroethyl)-3-oxoestra-4,9-dien-11 β-yl]benzaldehyde 1(E)-oxime
22) 4-[17β-hydroxy-17α-[(1,1,1-trifluoroethyloxy)methyl]-3-oxoestra-4,9-dien-11β-yl]benzaldehyde 1 (E)-oxime.

7. Process for the production of 17α-fluoroalkylated 11-β-benzaldoxime steroids with general formula I 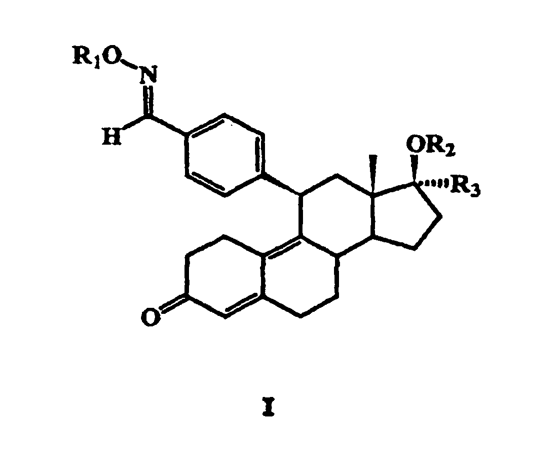 in which the radicals R₁, R₂ and R₃ have the meanings that are indicated in claims 1 to 5,
wherein an 11-β-benzaldehyde with the general formula II 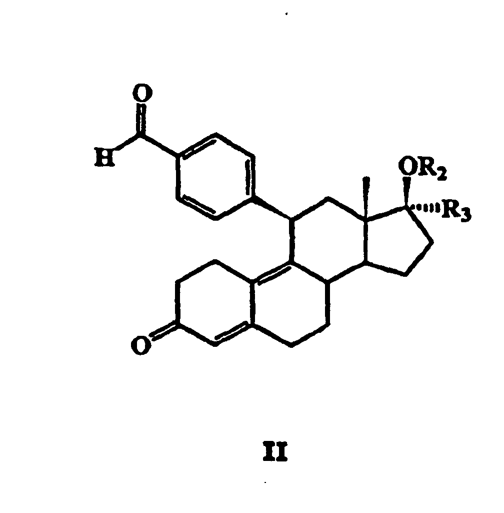 is reacted with a salt of a hydroxylamine in a basic solution, so that a 17α-fluoroalkyl-11β-benzaldoxime steroid is produced, in which R₁ is hydrogen, and this compound optionally is esterified, etherified or converted into a corresponding carbamate, carboxylic acid derivative or thiocarboxylic acid derivative.

8. Process according to claim 7, wherein the salt of the hydroxylamine is a hydrochloride or hydrosulfate.

9. Process according to one of claims 7 and 8, wherein the basic solvent is pyridine.

10. Pharmaceutical preparation containing at least one 17α-fluoroalkyl-11β-benzaldoxime steroid with general formula I according to one of claims 1 to 6 as well as at least one pharmaceutically compatible vehicle.

11. Use of the 17α-fluoroalkyl-11β-benzaldoxime steroids with general formula I according to one of claims 1 to 6 for the production of pharmaceutical agents.

## Revendications

1. 17α-Fluoroalkyl-11β-benzaldoxime-stéroïdes de formule générale I 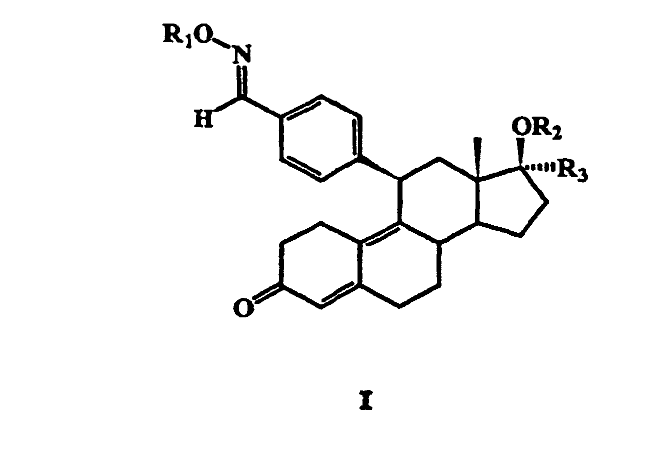 dans laquelle
R₁ représente un hydrogène, un alkyle en C₁ à C₆, COR₄, COOR₄, COSR₄ ou CONHR₅, dans lesquels R₄ est un alkyle en C₁ à C₆ ou un aryle non substitué ou substitué et R₅ est un hydrogène, un alkyle en C₁ à C₆ ou un aryle non substitué ou substitué,
R₂ représente un hydrogène, un alkyle en C₁ à C₆ ou un acyle en C₁ à C₆,
R₃ représente un groupement CₙF₂ₙ₊₁, dans lequel n = 1, 2 ou 3 ou un groupement CH₂O(CH₂)ₘCₙF₂ₙ₊₁, dans lequel m = 0 ou 1 et n = 1, 2 ou 3,
ainsi que leurs sels pharmaceutiquement compatibles.

2. 17α-Fluoroalkyl-11β-benzaldoxime-stéroïdes selon la revendication 1, **caractérisés en ce que** R₁ représente hydrogène, méthyl, formyl, acétyl, propionyl, benzoyl, méthoxycarbonyl, éthoxycarbonyl, méthylthiocarbonyl, éthylthiocarbonyl, éthylaminocarbonyl ou un phénylaminocarbonyl non substitué ou substitué.

3. 17α-Fluoroalkyl-11β-benzaldoxime-stéroïdes selon la revendication 2, **caractérisés en ce que** le radical phénylaminocarbonyl substitué est substitué avec un radical perfluoroalkyl en C₁ à C₆.

4. 17α-Fluoroalkyl-11β-benzaldoxime-stéroïdes selon une des revendications 1 à 3, **caractérisés en ce que** R₂ représente hydrogène, méthyl ou acétyl.

5. 17α-Fluoroalkyl-11β-benzaldoxime-stéroïdes selon une des revendications 1 à 4, **caractérisés en ce que** R₃ représente 1,1,1-trifluorométhyl, 1,1,2,2,2-pentafluoroéthyl, 1,1,2,2,3,3,3-heptafluoropropyl ou 1,1,1-trifluoroéthyloxymethyl.

6. 17α-Fluoroalkyl-11β-benzaldoxime-stéroïdes selon une des revendications 1 à 5, à savoir
1) 4-[17β-hydroxy-17α-(1,1,1-trifluorométhyl)-3-oxoestra-4,9-dién-11β-yl]-benzaldéhyde-1 (E)-oxime
2) 4-[17β-hydroxy-17α-(1,1,1-trifluorométhyl)-3-oxoestra-4,9-dién-11β-yl]-benzaldéhyde-1 (Z)-oxime
3) 4-[17β-hydroxy-17α-(1,1,1-trifluorométhyl)-3-oxoestra-4,9-dién-11β-yl]-benzaldéhyde-1 (E)-[O-(éthylamino)carbonyl]oxime
4) 4-[17β-hydroxy-17α-(1,1,2,2,2-pentafluoroéthyl)-3-oxoestra-4,9-dién-11 β-yl]-benzaldéhyde-1(E)-oxime
5) 4-[17β-hydroxy-17α-(1,1,2,2,2-pentafluoroéthyl)-3-oxoestra-4,9-dién-11 β-yl]-benzaldéhyde-1(E)-O-acétyloxime
6) 4-[17β-hydroxy-17α-(1,1,2,2,2-pentafluoroéthyl)-3-oxoestra-4,9-dién-11 β-yl]-benzaldéhyde-1(E)-[O-(éthylamino)carbonyl]oxime
7) 4-[17β-hydroxy-17α-(1,1,2,2,2-pentafluoroéthyl)-3-oxoestra-4,9-dién-11 β-yl]-benzaldéhyde-1(E)-[O-(éthylthio)carbonyl]oxime
8) 4-[17β-hydroxy-17α-(1,1,2,2,2-pentafluoroéthyl)-3-oxoestra-4,9-dién-11 β-yl]-benzaldéhyde-1(E)-[O-(éthoxy)carbonyl]oxime
9) 4-[17β-hydroxy-17α-(1,1,2,2,2-pentafluoroéthyl)-3-oxoestra-4,9-dién-11 β-yl]-benzaldéhyde-1(E)-[O-(méthoxy)carbonyl]oxime
10) 4-[17β-hydroxy-17α-(1,1,2,2,3,3,3-heptafluoropropyl)-3-oxoestra-4,9-dién-11 β-yl]-benzaldéhyde-1(E)-oxime
11) 4-[17β-méthoxy-17α-(1,1,1-trifluorométhyl)-3-oxoestra-4,9-dién-11β-yl]-benzaldéhyde-1 (E)-oxime
12) 4-[17β-méthoxy-17α-(1,1,1-trifluorométhyl)-3-oxoestra-4,9-dién-11β-yl]-benzaldéhyde-1 (E)-[O-(éthylamino)carbonyl]oxime
13) 4-[17β-méthoxy-17α-(1,1,2,2,2-pentafluoroéthyl)-3-oxoestra-4,9-dién-11 β-yl]-benzaldéhyde-1(E)-oxime
14) 4-[17β-méthoxy-17α-(1,1,2,2,2-pentafluoroéthyl)-3-oxoestra-4,9-dién-11 β-yl]-benzaldéhyde-1(E)-O-acétyloxime
15) 4-[17β-méthoxy-17α-(1,1,2,2,2-pentafluoroéthyl)-3-oxoestra-4,9-dién-11 β-yl]-benzaldéhyde-1(E)-[O-(éthylamino)carbonyl]oxime
16) 4-[17β-méthoxy-17α-(1,1,2,2,2-pentafluoroéthyl)-3-oxoestra-4,9-dién-11 β-yl]-benzaldéhyde-1(E)-{O-[(4'-trifluorométhoxy)phénylamino)]-carbonyl}oxime
17) 4-[17β-méthoxy-17α-(1,1,2,2,2-pentafluoroéthyl)-3-oxoestra-4,9-dién-11 β-yl]-benzaldéhyde-1(E)-[O-(éthoxy)carbonyl]oxime
18) 4-[17β-méthoxy-17α-(1,1,2,2,2-pentafluoroéthyl)-3-oxoestra-4,9-dién-11 β-yl]-benzaldéhyde-1(E)-[O-(méthoxy)carbonyl]oxime
19) 4-[17β-méthoxy-17α-(1,1,2,2,2-pentafluoroéthyl)-3-oxoestra-4,9-dién-11 β-yl]-benzaldéhyde-1(E)-[O-(éthylthio)carbonyl]oxime
20) 4-[17β-acétoxy-17α-(1,1,2,2,2-pentafluoroéthyl)-3-oxoestra-4,9-dién-11 β-yl]-benzaldéhyde-1(E)-[O-(éthylamino)carbonyl]oxime
21) 4-[17β-acétoxy-17α-(1,1,2,2,2-pentafluoroéthyl)-3-oxoestra-4,9-dién-11 β-yl]-benzaldéhyde-1(E)-oxime
22) 4-[17β-hydroxy-17α-[(1,1,1-trifluoroéthyloxy)méthyl]-3-oxoestra-4,9-dién-11 β-yl]-benzaldéhyde-1(E)-oxime.

7. Procédé de préparation de 11β-benzaldoxime-stéroïdes 17α-fluoroalkylés de formule générale I 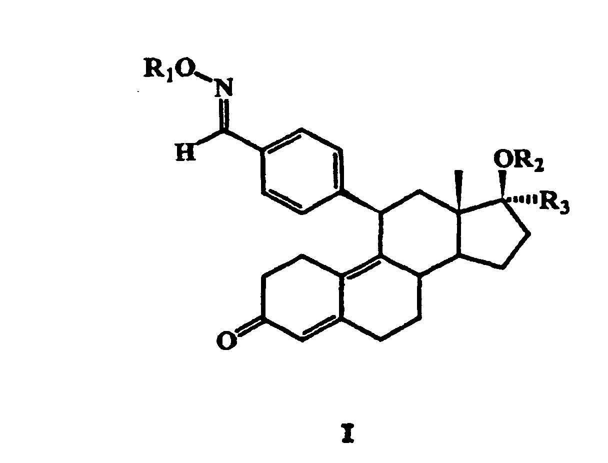 dans laquelle les radicaux R₁, R₂ et R₃ ont les significations indiquées dans les revendications 1 à 5,
**caractérisé en ce qu'**on met en réaction un 11β-benzaldéhyde de formule générale II 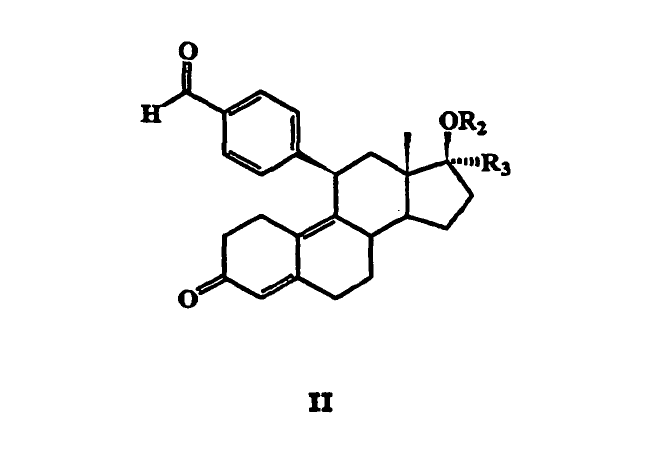 avec un sel d'un hydroxylamine dans un solvant basique de manière à former un 17α-fluoroalkyl-11β-benzaldoxime-stéroïde et qu'on estérifie, éthérifie ou transforme éventuellement ce composé en un carbamate, dérivé d'acide carbonique ou thiocarbonique correspondant.

8. Procédé selon la revendication 7, **caractérisé en ce que** le sel de l'hydroxylamine est un chlorhydrate ou un sulfhydrate.

9. Procédé selon une des revendications 7 et 8, **caractérisé en ce que** le solvant basique est de la pyridine.

10. Préparation pharmaceutique, contenant au moins un 17α-fluoroalkyl-11β-benzaldoxime-stéroïde de formule générale I selon une des revendications 1 à 6 ainsi qu'au moins un vecteur pharmaceutiquement compatible.

11. Utilisation de 17α-fluoroalkyl-11β-benzaldoxime-stéroïdes de formule générale I selon une des revendications 1 à 6 pour préparer des médicaments.
